# EUROPEAN PATENT SPECIFICATION

(11) **EP 1 713 437 B1**
(45) Date of publication and mention of the grant of the patent: **09.04.2008**
(21) Application number: 05722912.2
(22) Date of filing: 09.02.2005
(51) Int. Cl.: A61K 8/89, A61K 8/81, A61K 8/86, A61Q 5/12

(54) **CONDITIONING COMPOSITIONS COMPRISING HYDROPHOBICALLY MODIFIED CROSSLINKED CATIONIC THICKENING POLYMERS**
KONDITIONIERUNGSZUSAMMENSETZUNGEN MIT HYDROPHOBISCH MODIFIZIERTEN VERNETZTEN KATIONISCHEN VERDICKUNGSPOLYMEREN
COMPOSITIONS DE CONDITIONNEMENT COMPORTANT DES POLYMERES EPAISSISSANTS CATIONIQUES RETICULES A MODIFICATION HYDROPHOBE

(30) Priority: 10.02.2004 US 543397 P; 17.06.2004 US 580525 P
(43) Date of publication of application: 25.10.2006
(73) Proprietor: The Procter and Gamble Company, Cincinnati, Ohio 45202 (US)
(72) Inventor: ASARI, Satomi, Kobe, Hyogo 658-0054 (JP); GUSKEY, Susan Marie, Loveland, OH 45140 (US); WELLS, Robert, Lee, Cincinnati, Ohio 45229 (US); DECKNER, George, Endel, Cincinnati, Ohio 45249 (US)
(74) Representative: Engisch, Gautier
(86) International application number: PCT/US2005/004219
(87) International publication number: WO 2005/077323

(56) References cited:
- EP-A- 1 312 333
- WO-A-01/34103
- WO-A-01/76543
- US-A- 6 106 814

## Description

### FIELD OF THE INVENTION

The present invention relates to conditioning compositions comprising a hydrophobically modified crosslinked cationic thickening polymer, a surfactant system, and a silicone compound. The conditioning compositions of the present invention have a suitable rheology for conditioning compositions and provide conditioning benefits. The compositions are especially suitable for hair care products such as hair conditioning products for rinse-off use.

### BACKGROUND OF THE INVENTION

A variety of conditioning compositions such as hair conditioning compositions, skin conditioning compositions, and fabric softeners have been used for a variety of substrates such as hair, skin, and fabric. A common method of providing conditioning benefits is through the use of conditioning agents such as cationic surfactants and polymers, high melting point fatty compounds, low melting point oils, silicone compounds, and mixtures thereof. Most of these conditioning agents are known to provide various conditioning benefits. For example, some cationic surfactants, when used together with some high melting point fatty compounds, are believed to form a gel matrix which has a suitable rheology for conditioning compositions and which is suitable for providing a variety of conditioning benefits, especially when used for hair care products, such as slippery feel, softness and reduced tangling on wet hair and softness and moisturized feel on the dry hair.

There exists a need for achieving the suitable rheology for conditioning compositions by other methods than forming the above gel matrix, while maintaining the conditioning benefits of the gel matrix.

Additionally, most of the above conditioning agents are also known to make the composition opaque. Thus, there is a need for conditioning compositions having a clear product appearance i.e., transparent or translucent product appearance.

Furthermore, most of the above conditioning agents are also known to weigh down the hair when these conditioning agents are included in hair care compositions. For consumers who desire maintaining or increasing hair volume such as consumers having fine hair, weighing down the hair is not desirable. Thus, there is a need for hair conditioning compositions which do not weigh down the hair while providing conditioning benefits.

There also exists a need for conditioning compositions which consumers feel are easy to rinse-off while providing conditioning benefits when the compositions are used in a form of rinse-off products, especially rinse-off hair conditioner products. Conditioner compositions containing the above gel matrix also provide long-lasting slippery feel when rinsing which is not desirable for some consumers who prefer clean feel. Thus, there is a need for conditioning compositions which can easily leave the hair with a clean feel when rinsing the composition from the hair, while still depositing sufficient amount of conditioning agents on the hair.

Based on the foregoing, there remains a need for conditioning compositions which have a suitable rheology by other methods than a gel matrix comprised of cationic surfactants and high melting point fatty compounds, while providing conditioning benefits, especially softness and reduced tangling on wet hair, when used for hair care products such as hair conditioning products. There is also a need for such conditioning compositions which are suitable for providing further benefits such as clear product appearance, not weighing down the hair, and easy to rinse-off feel, while providing the above rheological and conditioning benefits.

None of the existing art provides all of the advantages and benefits of the present invention.

WO-A-01/34103 discloses rinse off hair conditioning compositions containing by weight:
(a) from 0.1 % to 5% of an hydrophobically modified cationic cellulose,
(b) from 0.01 % to 20% of a conditioning agent comprising cationic surfactant and
(c) silicone compounds and
(d) an aqueous carrier.

EP-A-1312333 discloses a rinse off hair conditioning compositions containing by weight:
(a) 0.2% of crosslinked cationic acryl polymer (Salcare SC 96™),
(b) 0.5% of non ionic surfactant hydrogenated polyoxyethylene castor oil (40 OE)
(c) 2% of polydimethylsiloxane DC2-8299™ and
(d) an aqueous carrier.

WO-A-01/76543 discloses rinse-off hair conditioning compositions containing by weight:
(a) 0.05-10% of a thickening system,
(b) 0.01-10% of a cationic surfactant,
(c) 0.1-40% of a silicone and
(d) an aqueous carrier.

US-A-6106814 discloses leave-on hair conditioning compositions containing by weight:
(a)/(c) from 0.1 % to 10% of cationic crosslinked conditioning polymeric agent which can be a polydimethylsiloxane gum,
(b) from 0.0001 % to 0.05% of polyethylene glycol and a cationic crosslinked acryl polymer and
(d) an aqueous carrier.

### SUMMARY OF THE INVENTION

The present invention is directed to compositions comprising by weight:
(a) from 0.01% to 6.0% of a hydrophobically modified crosslinked cationic thickening polymer;
(b) from 0.1% to 8.0% of a surfactant system according to any of the claims 1-3;
(c) from 0.1 % to 10% of a silicone compound; and
(d) an aqueous carrier.

These and other features, aspects, and advantages of the present invention will become better understood from a reading of the following description, and appended claims.

### DETAILED DESCRIPTION OF THE INVENTION

While the specification concludes with claims particularly pointing out and distinctly claiming the invention, it is believed that the present invention will be better understood from the following description.

Herein, "comprising" means that other steps and other ingredients which do not affect the end result can be added. This term encompasses the terms "consisting of" and "consisting essentially of".

All percentages, parts and ratios are based upon the total weight of the compositions of the present invention, unless otherwise specified. All such weights as they pertain to listed ingredients are based on the active level and, therefore, do not include carriers or by-products that may be included in commercially available materials.

Herein, "mixtures" is meant to include a simple combination of materials and any compounds that may result from their combination.

### COMPOSITIONS

The composition A of the present invention comprises by weight:
(a) from 0.01% to 6.0% of a hydrophobically modified crosslinked cationic thickening polymer;
(b) from 0.1% to 8.0% of a surfactant system comprising a cationic surfactant and a nonionic surfactant;
(c) from 0.1% to 10% of a silicone compound; and
(d) an aqueous carrier.

The composition B of the present invention comprises by weight:
(a) from 0.01% to 6.0% of a hydrophobically modified crosslinked cationic thickening polymer;
(b) from 0.1% to 8.0% of a surfactant system comprising nonionic surfactant having an HLB value of from 11 to 20;
(c) from 0.1% to 10% of a silicone compound; and
(d) an aqueous carrier.

The composition C of the present invention comprises by weight:
(a) from 0.01% to 6.0% of a hydrophobically modified crosslinked cationic thickening polymer;
(b) from 0.1% to 8.0% of a surfactant system selected from the group consisting of cationic surfactant, nonionic surfactant, and mixtures thereof;
(c) from 0.1% to 10% of a silicone compound, wherein the silicone compound is selected from those having an average particle size of 300nm or less in the composition, those being substantially soluble in the composition, and mixtures thereof, and wherein the substantially soluble silicone compound is selected from the group consisting of amino silicones, amino silicone copolyols, hydrophobically modified amino silicone copolyols, hydrophobically modified amido silicone copolyols, and mixtures thereof ; and
(d) an aqueous carrier.

The conditioning compositions A to C of the present invention have a suitable rheology for conditioning compositions and provide conditioning benefits, especially softness and reduced tangling on wet hair when used for hair care products such as hair conditioning products. Additionally, the conditioning compositions A to C of the present invention are suitable for providing further benefits such as clear product appearance, not weighing down the hair, and easy to rinse-off feel, while providing the above rheological and conditioning benefits. When used for hair care products, the compositions A to C of the present invention provide the above rheological and conditioning benefits while not weighing down the hair. Furthermore, when used in a form of rinse-off products, the compositions A to C of the present invention can provide easy to rinse-off feel while providing the above rheological and conditioning benefits.

It is believed that, by the combination of (a) the hydrophobically modified crosslinked cationic thickening polymer, (b) the surfactant system, and (c) the silicone compound, the compositions A to C of the present invention can provide a suitable rheology for conditioning compositions without the existence of a gel matrix comprised of cationic surfactants and high melting point fatty compounds, while providing conditioning benefits, especially softness and reduced tangling on wet hair when used for hair care products such as hair conditioning products. It is also believed that the preferable features of the present invention, such as the use of preferred thickening polymers, preferred surfactants and/or preferred silicone compounds, can help the compositions of the present invention to provide further benefits such as clear product appearance, not weighing down the hair, and easy to rinse-off feel, while providing the above rheological and conditioning benefits

Preferably, the compositions of the present invention are substantially free of water-insoluble oily compound selected from hydrocarbons, fatty compounds, and mixtures thereof. In the present invention, the compositions being "substantially free of water-insoluble oily compound" means that the composition includes 1.0% or less, preferably 0.5% or less, more preferably 0.1% or less, still more preferably 0% of water-insoluble oily compounds. The water-insoluble oily compounds herein are those having a solubility in water at 25°C of less than about 1g/100g water, preferably less than about 0.5g/100g water, more preferably less than about 0.1g/100g water. Such water-insoluble oily compounds are selected from hydrocarbons, fatty compounds, and mixtures thereof. Such hydrocarbons include, for example, poly α-olefin oils, paraffins, waxes, and mixtures thereof. Such fatty compounds include, for example, fatty alcohols such as cetyl alcohol and stearyl alcohol, fatty acids such as stearic acid, fatty alcohol derivatives and fatty acid derivatives such as esters and ethers thereof, and mixtures thereof.

Preferably the compositions of the present invention are transparent or translucent, and more preferably transparent. In the present invention, the composition being "transparent" means that the composition has a transmittance of 50% or more, preferably 65% or more, more preferably 80% or more. In the present invention, the compositions being "translucent" means that the compositions have a transmittance of from 25% to 50%, preferably from 35% to 50%. The transmittances are measured at 600nm using UV-1601 which is a UV-visible spectrophotometer available from Shimadzu.

Preferably, the compositions of the present invention are substantially free of anionic compounds. Anionic compounds herein include anionic surfactants and anionic polymers. In the present invention, the compositions being "substantially free of anionic compounds" means that the compositions include 1% or less, preferably 0.5% or less, more preferably 0% of anionic compounds.

### HYDROPHOBICALLY MODIFIED CROSSLINKED CATIONIC THICKENING POLYMER

The conditioning compositions of the present invention comprise a hydrophobically modified crosslinked cationic thickening polymer, preferably a hydrophobically modified crosslinked cationic acrylate thickening polymer. The cationic thickening polymers are included in the compositions of the present invention at a level by weight of from 0.01% to 6.0%, preferably from 0.1% to 5.0%, more preferably from 0.3% to 4.0%, still more preferably from 0.4% to 3.0%.

The hydrophobically modified crosslinked cationic thickening polymers useful herein are those which can provide appropriate viscosity and rheology properties to the composition, so that the compositions of the present invention have: (i) a suitable viscosity of preferably from 100 cps to 100,000 cps, more preferably from 1,000 cps to 50,000cps, still more preferably from 2,000cps to 50,000cps, even more preferably from 5,000 cps to 20,000cps; and (ii) suitable rheology properties such that the compositions have a Shear Thinning Index (STI) of preferably 30 or more, more preferably 50 or more, still more preferably 70 or more, even more preferably 90 or more. The viscosity herein can be suitably measured by Brookfield RVT at a shear rate of 2·s⁻¹ at 26.7°C. The Shear Thinning Index (STI) is calculated according to the following equation:

Shear Thinning Index (STI) = a first viscosity / a second viscosity; wherein the first viscosity is measured at a shear rate of 2·s⁻¹, and the second viscosity is measured at a shear rate of 950·s⁻¹, both at 26.7°C by shear rate ramp flow measurement using AR 2000 available from TA Instruments.

The hydrophobically modified crosslinked cationic thickening polymers useful herein may include the polymers disclosed below under the title "CATIONIC CONDITIONING POLYMER".

The hydrophobically modified crosslinked cationic thickening polymers useful herein are those being primarily cationic at the pH of the compositions of the present invention. Such polymers are those having an amine group selected from the group consisting of primary, secondary, tertiary, quaternary amines, and mixtures thereof. When the polymers have primary, secondary, and/or tertiary amines, the polymers are used in the compositions having a pH in which the amines are partially or completely protonated. Thus, when using such polymers having primary, secondary, and/or tertiary amines, the compositions preferably have a pH of from 2 to 8, more preferably from 3 to 7, still more preferably from 4 to 6.

The hydrophobically modified crosslinked cationic thickening polymers useful herein are those having a hydrophobic group. The hydrophobic group can be incorporated into the polymer as a hydrophobic substitution group attached to the polymeric backbone. The hydrophobic group useful herein is selected from a straight or branched, saturated or unsaturated chain alkyl group of preferably from 5 to 40 carbon atoms, more preferably from 8 to 35 carbon atoms, still more preferably from 10 to 30 carbon atoms.

Preferably, the hydrophobically modified crosslinked cationic thickening polymers useful herein have a hydrophilic group. The hydrophilic group can be incorporated into the polymer as a hydrophilic substitution group attached to the polymeric backbone. This hydrophilic group and the above hydrophobic group may be present in the same monomer unit or different monomer units. The hydrophilic groups useful herein include, for example, polyalkylene oxide groups containing from 1 to 50 alkylene oxides. The alkylene oxides are preferably selected from ethylene oxides, propylene oxides, and mixtures thereof.

Preferably, the hydrophobically modified crosslinked cationic thickening polymers useful herein is hydrophilic, and has a solubility in water at 25°C of at least 0.25g/100g water, more preferably at least 2g /100g water, still more preferably at least 5g/100g water, even more preferably at least 15g/100g water.

The polymeric backbones useful herein are those comprising synthetic monomers. Preferred synthetic monomers useful herein include, for example, vinyl monomers, acrylates monomers, methacrylate monomers, and mixtures thereof.

In the present invention, highly preferred are hydrophobically modified crosslinked cationic acrylate thickening polymers comprising the monomer units and have the formula_(A)ₘ(B)ₙ(C)ₚ(D)_{q}, wherein: (A) is an acrylate monomer; (B) is an amine-containing monomer; (C) is a hydrophilic monomer; (D) is a monomer having the hydrophobic substitution group; m, n, and q are independently one or greater, and p is zero or greater, preferably one or greater; and wherein the polymer further contain crosslinking groups.

The monomer (A) useful herein includes, for example, those selected from the group consisting of alkyl acrylate, alkyl methacrylate, acrylamide, methacrylamide, and mixtures thereof. Preferred are those selected from alkyl acrylate, alkyl methacrylate, or mixtures thereof. The alkyl portions of the monomer (A) are preferably short chain length alkyls such as C₁-C₈, more preferably C₁-C₅, still more preferably C₁-C₃, even still more preferably C₁-C₂.

The monomer (B) useful herein include, for example, those selected from the group consisting of a mono- or di-(C₁-C₄)alkylamino(C₁-C₄)alkyl(meth)acrylate, a mono- or di-(C₁-C₄)alkylamino(C₁-C₄)alkyl(meth)acrylamide, quaternized di-(C₁-C₄)alkylamino(C₁-C₄)alkyl (meth)acrylate, and mixtures thereof. Preferred are mono- or di-(C₁-C₄)alkylamino(C₁-C₄)alkyl(meth)acrylates. Exemplary monomers (b) include N,N-dimethylamino ethyl methacrylate (DMAEMA), N,N-diethylamino ethyl acrylate, N,N-diethylamino ethyl methacrylate, N-t-butylamino ethyl acrylate, N-t-butylamino ethyl methacrylate, N,N-dimethylamino propyl acrylamide, N,N-dimethylamino propyl methacrylamide, N,N-diethylamino propyl acrylamide, and N,N-diethylamino propyl methacrylamide.

The monomer (C) useful herein include, for example, those having polyalkylene oxide groups containing from 1 to 50 alkylene oxides, preferably from 5 to 45, more preferably from 10 to 40. The alkylene oxides are preferably selected from ethylene oxides, propylene oxides, and mixtures thereof. Preferred are those selected from the group consisting of vinyl alkoxylates, PEG 10-40 (meth)acrylates, and mixtures thereof.

The monomer (D) can be selected from any monomers having the hydrophobic group described above, i.e., straight or branched, saturated or unsaturated chain alkyl group of preferably from 5 to 40 carbon atoms, more preferably from 8 to 35 carbon atoms, still more preferably from 10 to 30 carbon atoms. Preferred monomer (D) further has a hydrophilic group in addition to the hydrophobic substitution group. Preferred hydrophilic groups are 10-40 ethylene glycols. Highly preferred monomer (D) has the above hydrophobic and hydrophilic groups in one substitution group, the substitution group attached to an ethylenically unsaturated copolymerizable backbone group such as acrylate and methacrylate. Even more preferred monomer (D) has the backbone group substituted with the hydrophobic group via the hydrophilic group.

In addition to required and preferred monomers discussed above, monomers which provide cross-linking in the polymer also may be utilized in relatively low amounts, preferably from 10ppm to 10,000ppm, more preferably from 10ppm to 1,000ppm, still more preferably from 10ppm to 250ppm, of the total weight of the polymer on a weight/weight basis. Cross-linking monomers include multi-vinyl-substituted aromatic monomers, multi-vinyl-substituted alicyclic monomers, di-functional esters of phthalic acid, di-functional esters of methacrylic acid, multi-functional esters of acrylic acid, N-methylene-bis-acrylamide and multi-vinyl-substituted aliphatic monomers such as dienes, trienes, and tetraenes. Exemplary cross-linking monomers include divinylbenzene, trivinylbenzene, 1,2,4-trivinylcyclohexane, 1,5-hexadiene, 1,5,9-decatriene, 1,9-decadiene, 1,5-heptadiene, di-allyl phthalate, ethylene glycol dimethacrylate, polyethylene glycol dimethacrylate, penta- and tetraacrylates, triallyl pentaerythritol, octaallyl sucrose, cycloparaffins, cycloolefins and N-methylene-bis-acrylamide.

Highly preferred hydrophobically modified crosslinked cationic acrylates thickening polymers useful herein include: those having an INCI name of Acrylates/Aminoacrylates/Vinylalkyoxylate/C₁₀₋₃₀ Alkyl PEG-25 Methacrylate copolymer, available from Noveon.

### SURFACTANT SYSTEM

The compositions of the present invention comprise a surfactant system according to any of the claims 1 to 3. The surfactant system is included in the compositions at a level by weight of from 0.1% to 8.0%, preferably from 0.2% to 5.0%, more preferably from 0.4% to 4.0%.

Preferably, in view of the desire for a transparent or translucent appearance, the surfactant system is substantially soluble in the compositions at the level used. By "substantially soluble" surfactant system, what is meant is that the compositions has a transmittance of 50% or more, preferably 65% or more, more preferably 80% or more at 25°C when containing the surfactant system at the level used.

The surfactant system useful herein is selected from the group of consisting of a cationic surfactant, a nonionic surfactant, and mixtures thereof. When contained, cationic surfactants are preferably used in combination with nonionic surfactants.

### Cationic surfactant

A variety of cationic surfactants including mono- and di-long alkyl chain cationic surfactants can be used in the compositions of the present invention as described below. Among them, preferred are mono-long alkyl chain cationic surfactants such as mono-long alkyl chain quaternary ammonium salts. The mono-long alkyl chain quaternary ammonium salts useful herein are those in which the mono-long alkyl chain has from 12 to 28 carbon atoms, preferably from 16 to 22 carbon atoms. Highly preferred mono-long alkyl chain quaternary ammonium salts are, for example, cetyl trimethyl ammonium chloride, stearyl trimethyl ammonium chloride. Although the mono-long alkyl chain cationic surfactants are preferred, other cationic surfactants such as di-long alkyl chain cationic surfactants may also be used alone, or in combination with the mono-long alkyl chain cationic surfactants and/or nonionic surfactants.

Cationic surfactants useful herein include, for example, those corresponding to the general formula (I): 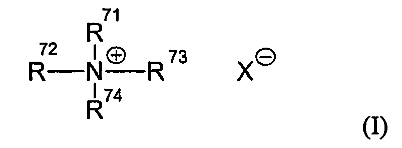 wherein at least one of R⁷¹, R⁷², R⁷³ and R⁷⁴ is selected from an aliphatic group of from 8 to 30 carbon atoms or an aromatic, alkoxy, polyoxyalkylene, alkylamido, hydroxyalkyl, aryl or alkylaryl group having up to about 22 carbon atoms, the remainder of R⁷¹, R⁷², R⁷³ and R⁷⁴ are independently selected from an aliphatic group of from 1 to about 22 carbon atoms or an aromatic, alkoxy, polyoxyalkylene, alkylamido, hydroxyalkyl, aryl or alkylaryl group having up to about 22 carbon atoms; and X is a salt-forming anion such as those selected from halogen, (e.g. chloride, bromide), acetate, citrate, lactate, glycolate, phosphate, nitrate, sulfonate, sulfate, alkylsulfate, and alkyl sulfonate radicals. The aliphatic groups can contain, in addition to carbon and hydrogen atoms, ether linkages, and other groups such as amino groups. The longer chain aliphatic groups, e.g., those of about 12 carbons, or higher, can be saturated or unsaturated. Preferred is when R⁷¹, R⁷², R⁷³ and R⁷⁴ are independently selected from C₁ to about C₂₂ alkyl.

Among the cationic surfactants of general formula (I), preferred are those containing in the molecule at least one alkyl chain having at least 16 carbons. Nonlimiting examples of such preferred cationic surfactants include: behenyl trimethyl ammonium chloride available, for example, with tradename Genamine KDMP from Clariant, with tradename INCROQUAT TMC-80 from Croda, and with tradename ECONOL TM22 from Sanyo Kasei; cetyl trimethyl ammonium chloride available, for example, with tradename CTAC 30KC from KCI, and with tradename CA-2350 from Nikko Chemicals; stearyl trimethyl ammonium chloride available, for example, with tradename Genamine STACP from Clariant; olealkonium chloride available, for example, with tradename Incroquat O-50 from Croda; hydrogenated tallow alkyl trimethyl ammonium chloride, dialkyl (14-18) dimethyl ammonium chloride, ditallow alkyl dimethyl ammonium chloride, dihydrogenated tallow alkyl dimethyl ammonium chloride, distearyl dimethyl ammonium chloride, and dicetyl dimethyl ammonium chloride.

Also preferred are hydrophilically substituted cationic surfactants in which at least one of the substituents contain one or more aromatic, ether, ester, amido, or amino moieties present as substituents or as linkages in the radical chain, wherein at least one of the R⁷¹-R⁷⁴ radicals contain one or more hydrophilic moieties selected from alkoxy (preferably C₁-C₃ alkoxy), polyoxyalkylene (preferably C₁-C₃ polyoxyalkylene), alkylamido, hydroxyalkyl, alkylester, and combinations thereof. Preferably, the hydrophilically substituted cationic conditioning surfactant contains from 2 to about 10 nonionic hydrophile moieties located within the above stated ranges. Highly preferred hydrophilically substituted cationic surfactants include dialkylamido ethyl hydroxyethylmonium salt, dialkylamidoethyl dimonium salt, dialkyloyl ethyl hydroxyethylmonium salt, dialkyloyl ethyldimonium salt, and mixtures thereof; for example, commercially available under the following tradenames: VARISOFT 110, ←: VARISOFT 222, VARIQUAT K1215 and VARIQUAT 638 from Witco Chemical, MACKPRO KLP, MACKPRO WLW, MACKPRO MLP, MACKPRO NSP, MACKPRO NLW, MACKPRO WWP, MACKPRO NLP, MACKPRO SLP from McIntyre, ETHOQUAD 18/25, ETHOQUAD O/12PG, ETHOQUAD C/25, ETHOQUAD S/25, and ETHODUOQUAD from Akzo, DEHYQUAT SP from Henkel, and ATLAS G265 from ICI Americas. Babassuamidopropalkonium Chloride available from Croda under the tradename Incroquat BA-85 is also preferably used in the composition.

Amines are suitable as cationic surfactants. Primary, secondary, and tertiary fatty amines are useful. Particularly useful are tertiary amido amines having an alkyl group of from about 12 to about 22 carbons. Exemplary tertiary amido amines include: stearamidopropyldimethylamine, stearamidopropyldiethylamine, stearamidoethyldiethylamine, stearamidoethyldimethylamine, palmitamidopropyldimethylamine, palmitamidopropyldiethylamine; palmitamidoethyldiethylamine, palmitamidoethyldimethylamine, behenamidopropyldimethylamine, behenamidopropyldiethylamine, behenamidoethyldiethylamine, behenamidoethyldimethylamine, arachidamidopropyldimethylamine, arachidamidopropyldiethylamine, arachidamidoethyldiethylamine, arachidamidoethyldimethylamine, diethylaminoethylstearamide. Useful amines in the present invention are disclosed in U.S. Patent 4,275,055, Nachtigal, et al. These amines can also be used in combination with acids such as ℓ-glutamic acid, lactic acid, hydrochloric acid, malic acid, succinic acid, acetic acid, fumaric acid, tartaric acid, citric acid, ℓ-glutamic hydrochloride, maleic acid, and mixtures thereof; more preferably ℓ-glutamic acid, lactic acid, citric acid. The amines herein are preferably partially neutralized with any of the acids at a molar ratio of the amine to the acid of from 1 : 0.3 to 1 : 2, more preferably from 1 : 0.4 to 1: 1.

Cationic surfactants can be included in the compositions at a level by weight of from 0.1 to 2.0%, preferably from 0.2% to 1.2%, more preferably from 0.4% to 1.0%.

### Nonionic surfactant

A variety of nonionic surfactants can be used in the compositions of the present invention. Non-limiting examples of nonionic surfactants include, for example, the following:
(1) polyethylene oxide condensates of alkyl phenols, e.g., the condensation products of alkyl phenols having an alkyl group containing from 6 to 20 carbon atoms in either a straight chain or branched chain configuration, with ethylene oxide, the said ethylene oxide being present in amounts equal to from 10 to 60 moles of ethylene oxide per mole of alkyl phenol;
(2) those derived from the condensation of ethylene oxide with the product resulting from the reaction of propylene oxide and ethylene diamine products;
(3) condensation products of aliphatic alcohols having from 8 to 18 carbon atoms, in either straight chain or branched chain configurations, with ethylene oxide, e.g., a coconut alcohol ethylene oxide condensate having from 10 to 30 moles of ethylene oxide per mole of coconut alcohol, the coconut alcohol fraction having from 10 to 14 carbon atoms;
(4) long chain tertiary amine oxides of the formula [R¹R²R³N → O] where R¹ contains an alkyl, alkenyl or monohydroxy alkyl radical of from 8 to 18 carbon atoms, from 0 to 10 ethylene oxide moieties, and from 0 to 1 glyceryl moiety, and R² and R³ contain from 1 to 3 carbon atoms and from 0 to 1 hydroxy group, e.g., methyl, ethyl, propyl, hydroxyethyl, or hydroxypropyl radicals;
(5) long chain tertiary phosphine oxides of the formula [RR'R"P → O] where R contains an alkyl, alkenyl or monohydroxyalkyl radical ranging from 8 to 18 carbon atoms in chain length, from 0 to 10 ethylene oxide moieties and from 0 to 1 glyceryl moieties and R' and R" are each alkyl or monohydroxyalkyl groups containing from 1 to 3 carbon atoms;
(6) long chain dialkyl sulfoxides containing one short chain alkyl or hydroxy alkyl radical of from 1 to 3 carbon atoms (usually methyl) and one long hydrophobic chain which include alkyl, alkenyl, hydroxy alkyl, or keto alkyl radicals containing from 8 to 20 carbon atoms, from 0 to 10 ethylene oxide moieties and from 0 to 1 glyceryl moieties;
(7) alkyl polysaccharide (APS) surfactants (e.g. alkyl polyglycosides), examples of which are described in U.S. Patent 4,565,647, and which discloses APS surfactants having a hydrophobic group with 6 to 30 carbon atoms and a polysaccharide (e.g., polyglycoside) as the hydrophilic group; optionally, there can be a polyalkylene-oxide group joining the hydrophobic and hydrophilic moieties; and the alkyl group (i.e., the hydrophobic moiety) can be saturated or unsaturated, branched or unbranched, and unsubstituted or substituted (e.g., with hydroxy or cyclic rings); a preferred material is alkyl polyglucoside, which is commercially available from Henkel, ICI Americas, and Seppic; and
(8) polyoxyethylene alkyl ethers such as those of the formula RO(CH₂CH₂O)ₙH and polyethylene glycol (PEG) glyceryl fatty esters, such as those of the formula R(O)OCH₂CH(OH)CH₂(OCH₂CH₂)ₙOH, wherein n is from 1 to 200, preferably from 20 to 100, and R is an alkyl having from 8 to 22 carbon atoms.

Polyethylene glycol derivatives of glycerides as described in the above (8) useful herein include derivatives of mono-, di- and tri-glycerides and mixtures thereof. One class of polyethylene glycol derivatives of glycerides suitable herein is those which conform to the general formula (I): 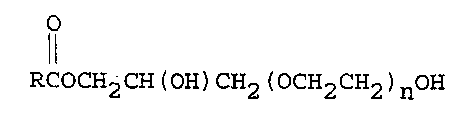 wherein n, the degree of ethoxylation, is from 4 to 200, preferably from 5 to 150, more preferably from 20 to 120, and wherein R comprises an aliphatic radical having from 5 to 25 carbon atoms, preferably from 7 to 20 carbon atoms. Suitable polyethylene glycol derivatives of glycerides can be polyethylene glycol derivatives of hydrogenated castor oil. Such polyethylene glycol derivatives of hydrogenated castor oil include, for example, PEG-20 hydrogenated castor oil, PEG-30 hydrogenated castor oil, PEG-40 hydrogenated castor oil, PEG-45 hydrogenated castor oil, PEG-50 hydrogenated castor oil, PEG-54 hydrogenated castor oil, PEG-55 hydrogenated castor oil, PEG-60 hydrogenated castor oil, PEG-80 hydrogenated castor oil, and PEG-100 hydrogenated castor oil.

Other suitable polyethylene glycol derivatives of glycerides can be polyethylene glycol derivatives of stearic acid. Such polyethylene glycol derivatives of stearic acid include, for example, PEG-30 stearate, PEG-40 stearate, PEG-50 stearate, PEG-75 stearate, PEG-90 stearate, PEG-100 stearate, PEG-120 stearate, and PEG-150 stearate.

Ethylene glycol ethers of fatty alcohols, as described in the above (3) or (8), useful herein include any ethylene glycol ethers of fatty alcohols which are suitable for use in a hair conditioning composition. No limiting examples of the ethylene glycol ethers of fatty alcohols include; the ceteth series of compounds such as ceteth-1 through ceteth-45, preferably ceteth-7 through ceteth-20; the isoceteth series of compounds such as isoceteth-20; the steareth series of compounds such as steareth-1 through 100; ceteareth 1 through ceteareth-50; the laureth series of compounds, preferably laureth-7 through Laureth-12; the pareth series of compounds, preferably pareth-9 through pareth-15; propylene glycol ethers of the above ceteth, steareth, ceteareth, and laureth series of compounds, such propylene glycol ethers of ceteth series of compounds including, for example, PPG-5-Ceteth-20; polyoxyethylene ethers or polyoxyethylene-polyoxypropylene ethers of branched alcohols, such branched alcohols including, for example, octyldodecyl alochol, decyltetradecyl alcohol, dodecylpentadecyl alcohol, hexyldecyl alcohol, and isostearyl alcohol, and such polyoxyethylene-polyoxypropylene ethers of branched alcohols including, for example, POE(20)POP(6) decyltetradecyl ether; and mixtures thereof.

Other nonionic surfactants useful herein include, for example, polysorbates such as polysorbate-20 (POE(20) sorbitan monolaurate) having HLB value of 16.7, polysorbate-21 (POE(4) sorbitan monolaurate) having HLB value of 13.3, polysorbate-40 (POE(20) sorbitan monopalmitate) having HLB value of 15.6, polysorbate-60 (POE(20) sorbitan monostearate) having HLB value of 14.9, polysorbate-61 (POE(4) sorbitan monostearate) having HLB value of 9.6, polysorbate-80 (POE(20)sorbitan monooleate) having HLB value of 15.0, and polysorbate-81 (POE(4) sorbitan monooleate) having HLB value of 10.0.

Preferably, the nonionic surfactants useful herein have an HLB value of from 8 to 22, more preferably from 11 to 20, still preferably from 13 to 15.

Among a variety of nonionic surfactants described above, highly preferred are those selected from the group consisting of isoceteth-20, PPG-5-Ceteth-20, PEG-40 hydrogenated castor oil, polysorbate-20, laureth-20, ceteth-10, ceteth-20, and mixtures thereof.

Nonionic surfactants can be included in the compositions at a level by weight of from 0.1 to 6.0%, preferably from 0.4% to 5.0%, more preferably from 1.0% to 4.0%.

### SILICONE COMPOUND

The compositions of the present invention comprise a silicone compound. The silicone compound is included in the compositions at levels by weight of from 0.1% to 10%, more preferably from 0.5% to 8%, still more preferably from 1% to 6%, even more preferably from 2% to 5%.

Among a variety of silicone compounds, preferred are those selected from the group consisting of (i) silicone nanoemulsion having an average particle size of 300nm or less, preferably 200nm or less, more preferably 100nm or less when contained in the composition, (ii) silicone compound being substantially soluble in the composition, and (iii) mixtures thereof, in view of the desire for a transparent or translucent appearance. By "substantially soluble" silicone compound, what is meant is that the compositions having a transmittance of 50% or more, preferably 65% or more, more preferably 80% or more at 25°C when containing the silicone compound at the level used.

Commercially available silicone nanoemulsion useful herein includes, for example, that with a tradename Silicone DC-8177 available from Dow Coming; quaternized silicone nanoemulsion with a tradename DC5-7133 available from Dow Coming; and amodimethicone nanoemulsion with a tradename XS65-B6413 available from General Electric.

With respect to substantially soluble silicone compounds, for example, following materials can be substantially soluble depending on the level of hydrophilic groups in their structure: silicone copolyols such as dimethicone copolyol with a tradename Silicone DC-5330 from Dow Coming; amino silicones such as those having a amine content which is high enough to make the amino silicones substantially soluble; amino silicone copolyols such as those having an INCI name Bis (C13-15 Alkoxy) PG Amodimethicone available with a tradename DC2-8500 from Dow Coming; hydrophobically modified amino silicone copolyols; hydrophobically modified amido silicone copolyols; and quaternized silicones. Among these substantially soluble silicone compounds, more preferred are those selected from the group consisting of amino silicones, amino silicone copolyols, hydrophobically modified amino silicone copolyols, and hydrophobically modified amido silicone copolyols, and mixtures thereof, still more preferred are hydrophobically modified amido silicone copolyols in view of providing improved silicone deposition thus providing improved conditioning performance while meeting the desire for transparent or translucent appearance. Such hydrophobically modified amidomethicone copolyols have the general formula: 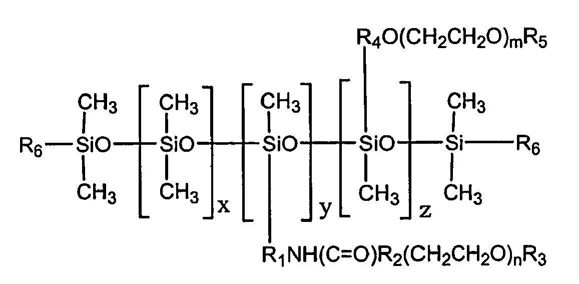 wherein R₁, R₂, R₄ are respectively C1-C3 alkyl, preferably ethyl; R₃ is an alkyl group having 8-22 carbon atoms, preferably 10-20 carbon atoms, more preferably 12-16 carbon atoms, even more preferably 12 carbon atoms; R₅ is H or C1-C3 alkyl, preferably methyl; R₆ is OH or CH₃, preferably methyl; n is an integer of 1-10, highly preferably 5; m is an integer of 2-20, highly preferably 12; n+m = 3-30, preferably 5-25, more preferably 8-20, even more preferably 17; x is an integer from 200 to 500, preferably from 300 to 400; y is an integer from 5 to 40, preferably from 10 to 30; and z is 0 or an integer from 1 to 30, preferably from 5 to 20. Commercially available hydrophobically modified amido silicone copolyols are, for example, those having an_INCI name PEG-12 Methyl Ether/Lauroxy PEG-5 Amidopropyl Dimethicone available with a tradename Silicone BY16-906 from Dow Corning.

In view of providing improved conditioning benefits, it is preferred for the compositions of the present invention to provide improved silicone deposition, even after rinsing-off the compositions from the hair. For example, it is preferred for the compositions to provide silicone deposition of 50ppm or more, more preferably 100ppm or more, still more preferably 200ppm or more, even more preferably 400ppm or more after rinsing-off the hair. The amount of the silicone deposition can be measured by a method consisting of: (i) a preparation of hair switch; and (ii) silicone deposition measurement.

### (i) Preparation of hair switch

For the silicone deposition measurement, 2 gram hair switches are used. The hair switches are prepared by following steps:
(a) Providing five cycles of shampoo/conditioning treatments to the hair switch, each cycle of shampoo/conditioning treatment consisting of following steps:
   (a-1) Applying a shampoo at a level of 0.2cc and lathering the hair switch; and rinsing the hair switch;
   (a-2) Applying a shampoo again at a level of 0.2cc and lathering the hair switch; and rinsing the hair switch; and
   (a-3) Then providing conditioner treatment to the hair switch, the conditioner treatment consisting of applying a conditioner at a level of 0.2cc and treating the hair switch; and rinsing the hair switch; and
(b) Then drying the hair switch.
The hair switch is ready for the measurement of its silicone deposition amount.

### (ii) Silicone deposition measurement

The deposited silicone on the hair switch is extracted in an appropriate solvent. The extracts are then introduced into an atomic absorption/emission detector instrument and measured at the appropriate wavelength. The absorbance/emission value returned by the instrument is then converted to actual concentration (ppm) of silicone compound deposited on the hair through an external calibration curve obtained with known weights of a well characterized standard of the silicone compound under study.

### AQUEOUS CARRIER

The compositions of the present invention comprise an aqueous carrier. The level and species of the carrier are selected according to the compatibility with other components, and other desired characteristic of the product.

Carriers useful in the present invention include water and water solutions of lower alkyl alcohols. Lower alkyl alcohols useful herein are monohydric alcohols having 1 to 6 carbons, more preferably ethanol and isopropanol.

Preferably, the aqueous carrier is substantially water. Deionized water is preferably used. Water from natural sources including mineral cations can also be used, depending on the desired characteristic of the product. Generally, the compositions of the present invention comprise from 20% to 99%, preferably from 40% to 98%, and more preferably from 50% to 98% water.

The pH of the present compositions are preferably from 2 to 8, more preferably from 3 to 7, still more preferably from 4 to 6. Buffers and other pH adjusting agents can be included to achieve the desirable pH.

### ADDITIONAL COMPONENTS

The compositions of the present invention may include additional components, which may be selected by the artisan according to the desired characteristics of the final product and which are suitable for rendering the compositions more cosmetically or aesthetically acceptable or to provide them with additional usage benefits.

### Cationic conditioning polymer

The conditioning compositions of the present invention may further include cationic conditioning polymers. The cationic polymers hereof will generally have a weight average molecular weight which is at least 5,000, typically at least 10,000, and is less than 10 million, preferably, the molecular weight is from 100,000 to 2 million.

The cationic conditioning polymer can be included in the compositions at a level by weight of preferably from 0.01% to 10%, more preferably from 0.05% to 5%.

Suitable cationic conditioning polymers include, for example: copolymers of 1-vinyl-2-pyrrolidone and 1-vinyl-3-methylimidazolium salt (e.g., chloride salt) (referred to in the industry by the Cosmetic, Toiletry, and Fragrance Association, "CTFA", as Polyquaternium-16), such as those commercially available from BASF Wyandotte Corp. (Parsippany, NJ, USA) under the LUVIQUAT tradename (e.g., LUVIQUAT FC 370); copolymers of 1-vinyl-2-pyrrolidone and dimethylaminoethyl methacrylate (referred to in the industry by CTFA as Polyquaternium-11) such as those commercially available from Gaf Corporation (Wayne, NJ, USA) under the GAFQUAT tradename (e.g., GAFQUAT 755N); cationic diallyl quaternary ammonium-containing polymers, including, for example, dimethyldiallylammonium chloride homopolymer and copolymers of acrylamide and dimethyldiallylammonium chloride, referred to in the industry (CTFA) as Polyquaternium 6 and Polyquaternium 7, Polyquaternium-7 including that commercially available with a tradename Merquat 550 from Ondeo Nalco; polymethacrylamidopropyl trimonium chloride such as that commercially available with a tradename Polycare 133 from Rhone-Poulenc; and Polyquaternium-37 available from 3V Sigma with tradenames Synthalen CR, Synthalen CU, and Synthalen CN.

Also suitable cationic conditioning polymers herein include cationic cellulose derivatives. Cationic cellulose derivative useful herein include, for example, salts of hydroxyethyl cellulose reacted with trimethyl ammonium substituted epoxide, referred to in the industry (CTFA) as Polyquaternium 10, available from Amerchol Corp. (Edison, NJ, USA) in their Polymer JR^{®} and LR^{®} series, and also available from National Starch & Chemical with a tradename Celquat SC-230M; and Polyquaternium-4 with tradename Celquat H-100 available from National Starch & Chemical.

Other suitable cationic conditioning polymers include cationic guar gum derivatives, such as guar hydroxypropyltrimonium chloride commercially available from Rhodia in their Jaguar series.

### Humectant and/or Co-solvent

The compositions of the present invention may contain a humectant and/or co-solvent to help the surfactant system and/or silicone compound to be substantially soluble in the composition. The humectants and/or co-solvents herein are selected from the group consisting of polyhydric alcohols, water soluble alkoxylated nonionic polymers, and mixtures thereof. The humectants and/or co-solvents herein are preferably used at levels by weight of the compositions of from 0.1 % to 20%, more preferably from 0.5% to 5%.

Polyhydric alcohols useful herein include glycerin, sorbitol, propylene glycol, butylene glycol, hexylene glycol, ethoxylated glucose, 1, 2-hexane diol, hexanetriol, dipropylene glycol, erythritol, trehalose, diglycerin, xylitol, maltitol, maltose, glucose, fructose, sodium chondroitin sultate, sodium hyaluronate, sodium adenosin phosphate, sodium lactate, pyrrolidone carbonate, glucosamine, cyclodextrin, and mixtures thereof. Among them, preferred for the co-solvents are 1,2-hexane diol, hexylene glycol, butylene glycol, glycerine, and mixtures thereof.

Water soluble alkoxylated nonionic polymers useful herein include polyethylene glycols and polypropylene glycols having a molecular weight of up to 10,000 such as those with CTFA names PEG-4, PEG-8, PEG-12, PEG-20, PEG-150 and mixtures thereof.

### Other additional components

The compositions of the present invention may further include other additional components. Other additional components generally are used individually at levels of from 0.001% to 10%, preferably up to 5% by weight of the composition.

A wide variety of other additional components can be formulated into the present compositions. These include: other conditioning agents such as hydrolysed collagen with tradename Peptein 2000 available from Hormel, vitamin E with tradename Emix-d available from Eisai, panthenol available from Roche, panthenyl ethyl ether available from Roche, nonionic surfactants such as glyceryl stearate available from Stepan Chemicals, hydrolysed keratin, proteins, plant extracts, and nutrients; emollients such as PPG-3 myristyl ether with tradename Varonic APM available from Goldschmidt, Trimethyl pentanol hydroxyethyl ether, PPG-11 stearyl ether with tradename Varonic APS available from Goldschmidt, Stearyl heptanoate with tradename Tegosoft SH available from Goldschmidt, Lactil (mixture of Sodium lactate, Sodium PCA, Glycine, Fructose, Urea, Niacinamide, Inositol, Sodium Benzoate, and Lactic acid) available from Goldschmidt, Ethyl hexyl palmitate with tradename Saracos available from Nishin Seiyu and with tradename Tegosoft OP available from Goldschmidt; hair-fixative polymers such as amphoteric fixative polymers, cationic fixative polymers, anionic fixative polymers, nonionic fixative polymers, and silicone grafted copolymers; preservatives such as benzyl alcohol, methyl paraben, propyl paraben and imidazolidinyl urea; pH adjusting agents, such as citric acid, sodium citrate, succinic acid, phosphoric acid, sodium hydroxide, sodium carbonate; salts, in general, such as potassium acetate and sodium chloride; coloring agents, such as any of the FD&C or D&C dyes; hair oxidizing (bleaching) agents, such as hydrogen peroxide, perborate and persulfate salts; hair reducing agents such as the thioglycolates; perfumes; and sequestering agents, such as disodium ethylenediamine tetra-acetate; ultraviolet and infrared screening and absorbing agents such as octyl salicylate; antidandruff agents such as zinc pyrrithione and salicylic acid; visible particles with tradenames Unisphere and Unicerin available from Induchem AG (Switzerland); and anti-foaming agent such as that with a tradename XS63-B8929 available from GE-Toshiba Silicone.

### PRODUCT FORMS

The conditioning compositions of the present invention can be in the form of rinse-off products or leave-on products, can be transparent, translucent, or opaque, and can be formulated in a wide variety of product forms, including but not limited to creams, gels, emulsions, mousses and sprays.

The conditioning compositions of the present invention can be used for conditioning a variety of substrates such as hair, skin, and fabric, by applying the compositions to the substrates such as hair, skin, and fabric. The conditioning compositions of the present invention is especially suitable for hair care products such as hair conditioners, skin care products such as skin conditioners, and fabric care products such as fabric softeners.

The conditioning compositions of the present invention are especially suitable for hair conditioners for rinse-off use. Such compositions are preferably used by following steps:
(i) after shampooing hair, applying to the hair an effective amount of the conditioning compositions for conditioning the hair; and
(ii) then rinsing the hair.

### EXAMPLES

The following examples further describe and demonstrate embodiments within the scope of the present invention. The examples are given solely for the purpose of illustration and are not to be construed as limitations of the present invention, as many variations thereof are possible without departing from the spirit and scope of the invention. Ingredients are identified by chemical or CTFA name, or otherwise defined below.

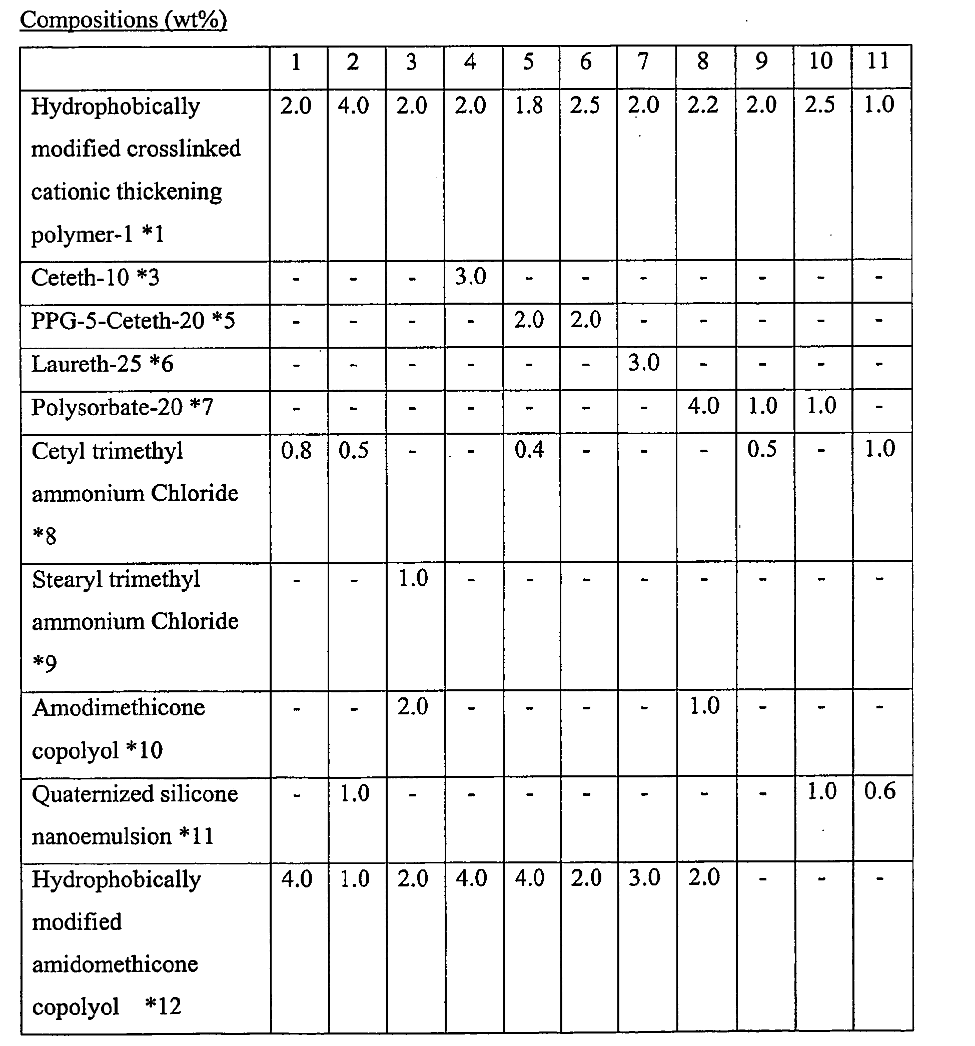

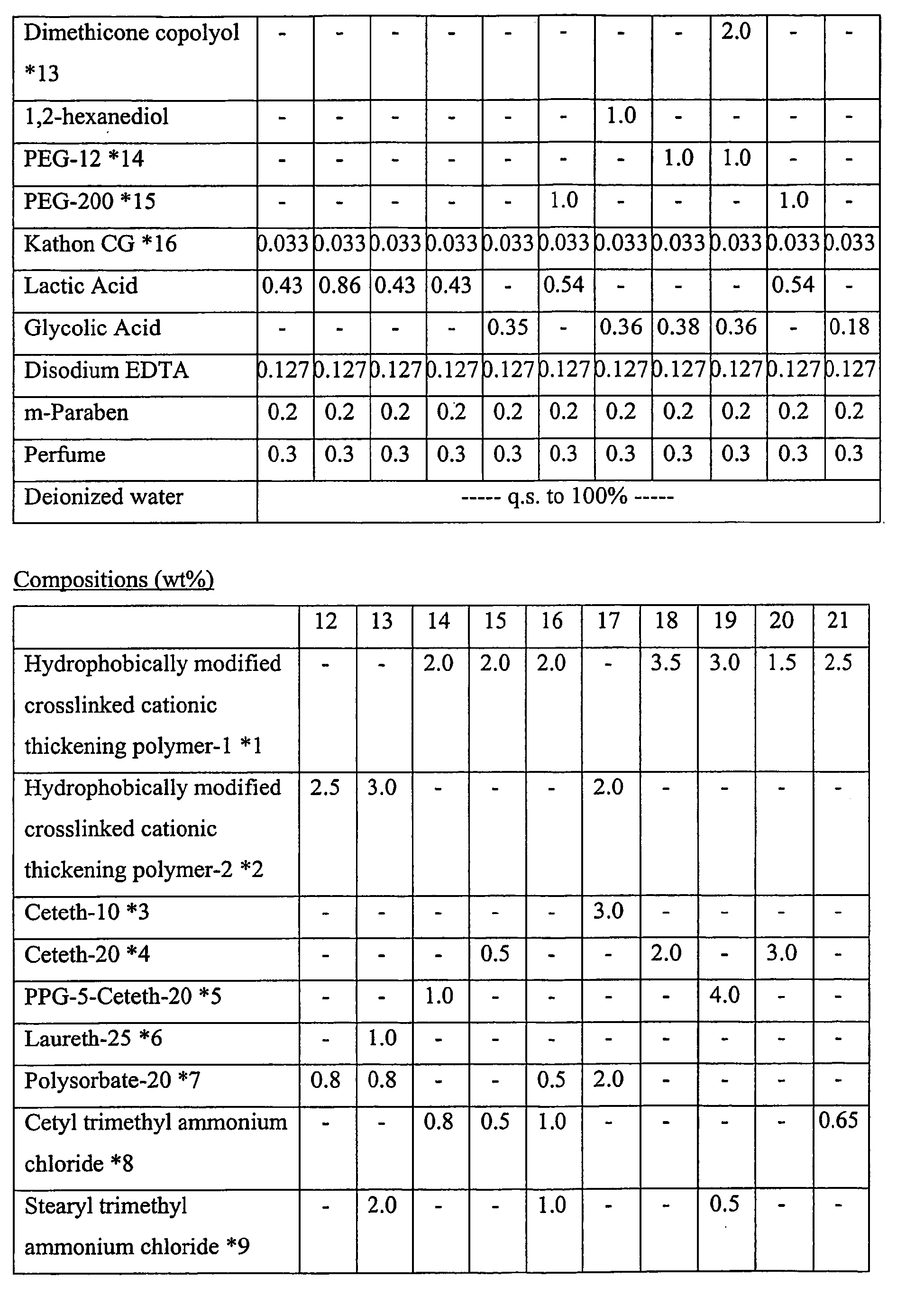

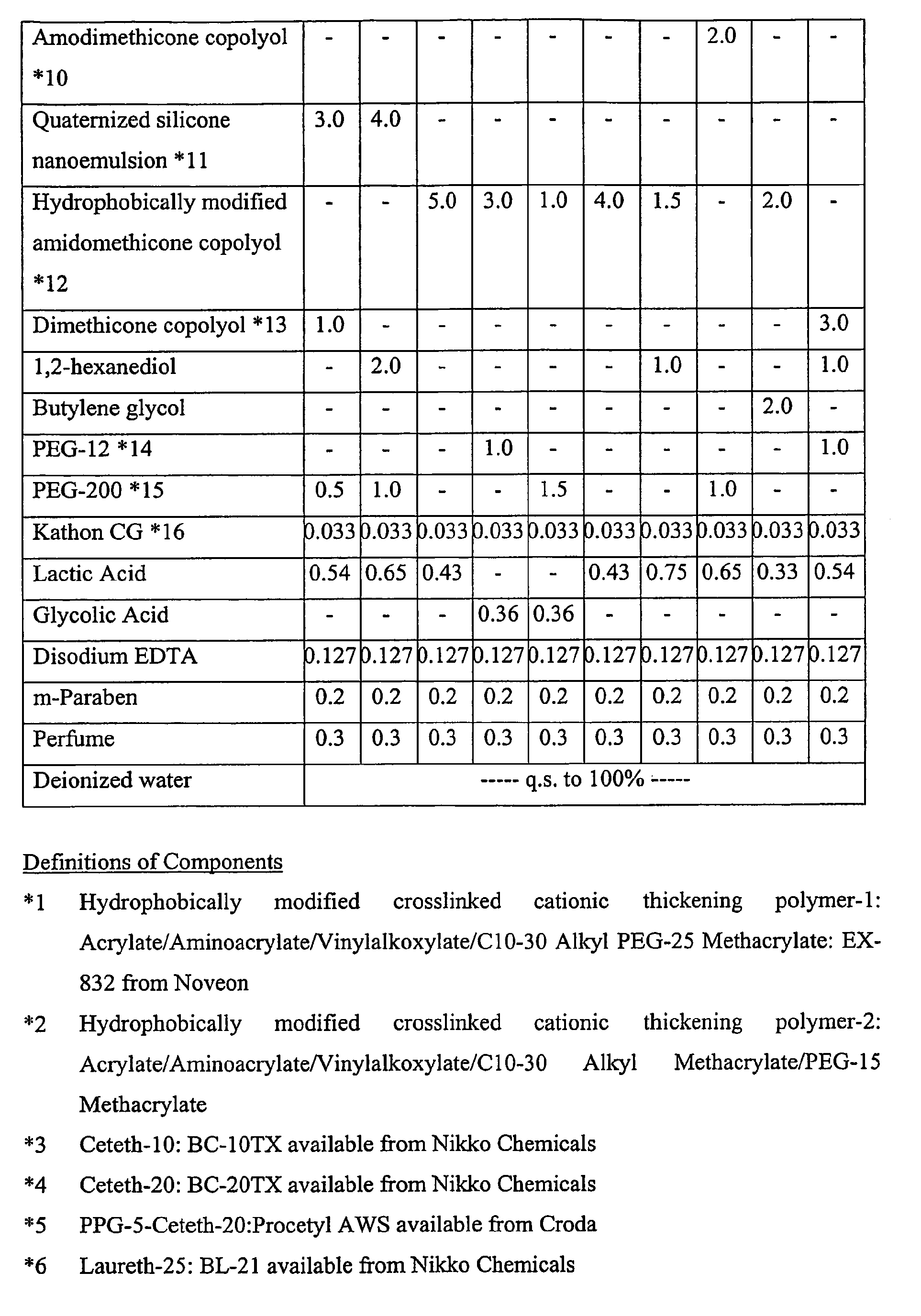

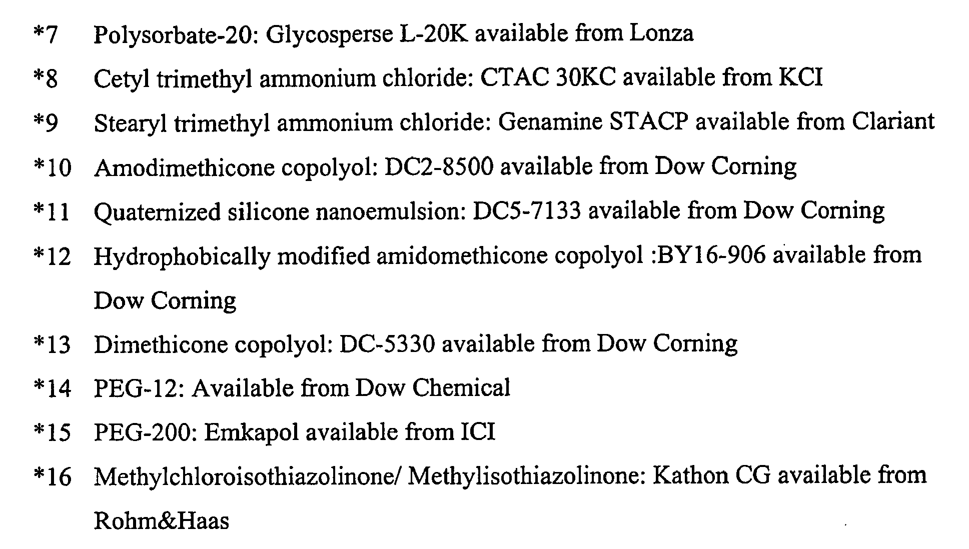

### Method of preparation

The conditioning compositions of "Ex.1" to "Ex.21" as shown above can be prepared by any conventional method well known in the art. They are suitably made as follows:

The polymeric materials are dispersed in water at room temperature, mixed with vigorous agitation. Separately, silicone compounds, surfactant system, and if included, other oily components such as perfumes are mixed at room temperature. The mixture containing silicone compounds and surfactant system is added to the polymeric dispersion with agitation. Then, the remaining components such as preservatives, anti-foaming agents, and pH adjusting agents, are added to the mixture with agitation.

Examples 1 through 21 are conditioning compositions of the present invention which are particularly useful for hair conditioners for rinse-off use. These examples have many advantages. For example, the compositions of "Ex.1" through "Ex.21" have a suitable rheology for conditioning compositions, and provide conditioning benefits, especially softness and reduced tangling on wet hair when used for hair care products such as hair conditioning products. The compositions of "Ex.1" and "Ex.4" have transparent or translucent appearance. When used for hair care products, the compositions of "Ex.1" through "Ex.21" can provide the above rheological and conditioning benefits while not weighing down the hair. When used in a form of rinse-off products, the compositions of "Ex.1" through "Ex.21" can provide easy to rinse-off feel while providing the above rheological and conditioning benefits.

## Claims

1. A conditioning composition comprising by weight:
(a) from 0.01% to 6.0% of a hydrophobically modified crosslinked cationic thickening polymer;
(b) from 0.1% to 8.0% of a surfactant system comprising a cationic surfactant and a nonionic surfactant;
(c) from 0.1 % to 10% of a silicone compound; and
(d) an aqueous carrier.

2. A conditioning composition comprising by weight:
(a) from 0.01% to 6.0% of a hydrophobically modified crosslinked cationic thickening polymer;
(b) from 0.1% to 8.0% of a surfactant system comprising nonionic surfactant having an HLB value of from about 11 to about 20;
(c) from 0.1 % to 10% of a silicone compound; and
(d) an aqueous carrier.

3. A conditioning composition comprising by weight:
(a) from 0.01% to 6.0% of a hydrophobically modified crosslinked cationic thickening polymer;
(b) from 0.1% to 8.0% of a surfactant system selected from the group consisting of cationic surfactant, nonionic surfactant, and mixtures thereof;
(c) from 0.1% to 10% of a silicone compound, wherein the silicone compound is selected from those having an average particle size of 300nm or less in the composition, those being substantially soluble in the composition, and mixtures thereof, and wherein the substantially soluble silicone compound is selected from the group consisting of amino silicones, amino silicone copolyols, hydrophobically modified amino silicone copolyols, hydrophobically modified amido silicone copolyols, and mixtures thereof ; and
(d) an aqueous carrier.

4. The conditioning composition of any of Claims 1 to 3 wherein the composition is substantially free of a water-insoluble oily compound selected from hydrocarbons, fatty compounds, and mixtures thereof.

5. The conditioning composition of any of Claims 1 to 3 wherein the composition is transparent or translucent.

6. The conditioning composition of Claim 5 wherein the composition is transparent.

7. The conditioning composition of Claim 6 wherein the composition has a transmittance of 50% or more.

8. The conditioning composition of Claim 7 wherein the composition has a transmittance of 65% or more.

9. The conditioning composition of Claim 8 wherein the composition has a transmittance of 80% or more.

10. The conditioning composition of any of Claims 1 to 3 wherein the composition has a viscosity of from 100cps to 100,000cps and Shear Thinning Index of 30 or more.

11. The conditioning composition of Claim 10 wherein the composition has a viscosity of from 1,000cps to 50,000cps and Shear Thinning Index of 50 or more.

12. The conditioning composition of Claim 11 wherein the composition has a viscosity of from 2,000cps to 50,000cps and Shear Thinning Index of 70 or more.

13. The conditioning composition of Claim 12 wherein the composition has a viscosity of from 5,000cps to 20,000cps and Shear Thinning Index of 90 or more.

14. The conditioning composition of any of Claims 1 to 3 wherein the hydrophobically modified crosslinked cationic thickening polymer is a hydrophobically modified crosslinked cationic acrylate thickening polymer.

15. The conditioning composition of any of Claims 1 to 3 wherein the hydrophobically modified crosslinked cationic thickening polymer has a hydrophilic group.

16. The conditioning composition of Claim 15 wherein the hydrophilic group is a polyalkylene oxide group.

17. The conditioning composition of Claim 16 wherein the alkylene oxide is selected from the group consisting of ethylene oxide, propylene oxide, and mixtures thereof.

18. The conditioning composition of any of Claims 1 to 3 wherein the surfactant system is substantially soluble in the composition.

19. The conditioning composition of Claim 1 or 3 wherein the nonionic surfactant has an HLB value of from 8 to 22.

20. The conditioning composition of either Claim 1 or 3, wherein the nonionic surfactant is selected from the group consisting of isoceteth-20, PPG-5-Ceteth-20, PEG-40 hydrogenated castor oil, polysorbate-20, laureth-20, ceteth-10, ceteth-20, and mixtures thereof.

21. The conditioning composition of Claim 1 or 3 wherein the cationic surfactant is selected from the group consisting of mono-long alkyl quaternary ammonium salts, di-long alkyl quaternary ammonium salts, hydrophilically substituted mono-long alkyl quaternary ammonium salts, hydrophilically substituted di-long alkyl quaternary ammonium salts, mono-long alkyl chain amines, di-alkyl chain amines, and mixtures thereof.

22. The conditioning composition of Claim 21 wherein the cationic surfactant is selected from the group consisting of cetyl trimethyl ammonium chloride, stearyl trimethyl ammonium chloride, di-cetyl dimethyl ammonium chloride, and mixtures thereof.

23. The conditioning composition of Claim 1 or 2 wherein the silicone compound is selected from those having an average particle size of 300nm or less in the composition, those being substantially soluble in the composition, and mixtures thereof.

24. The conditioning composition of Claim 23 wherein the substantially soluble silicone compound is selected from the group consisting of amino silicones, amino silicone copolyols, hydrophobically modified amino silicone copolyols, hydrophobically modified amido silicone copolyols, and mixtures thereof.

25. The conditioning composition of Claim 24 wherein the silicone compound is a hydrophobically modified amido silicone copolyol.

26. The conditioning composition of any of Claims 1 to 3 further comprising a co-solvent.

27. The conditioning composition of Claim 26 wherein the co-solvent is selected from the group consisting of 1,2-hexane diol, hexylene glycol, butylene glycol, glycerine, and mixtures thereof.

28. The conditioning composition of any of Claims 1 to 3 comprising by weight:
(a) from 0.1% to 5.0% of the hydrophobically modified crosslinked cationic thickening polymer;
(b) from 0.2% to 5.0% of the surfactant system according any of claims 1 to 3;
(c) from 0.5% to 8.0% of the silicone compound; and
(d) an aqueous carrier.

29. The conditioning composition of Claim 28 comprising by weight:
(a) from 0.3% to 4.0% of the hydrophobically modified crosslinked cationic thickening polymer;
(b) from 0.4% to 4.0% of the surfactant system according to any of claims 1 to 3;
(c) from 1.0% to 6.0% of the silicone compound; and
(d) an aqueous carrier.

30. The conditioning composition of any of Claims 1 to 3 which is a hair conditioning composition.

31. The conditioning composition of any of Claims 1 to 3 which is for rinse-off use.

32. The conditioning composition of any of Claims 1 to 3 which provides silicone deposition of about 50ppm or more after rinsed off from the hair.

33. The conditioning composition of Claim 32 which provides silicone deposition of about 100ppm or more after rinsed off from the hair.

34. The conditioning composition of Claim 33 which provides silicone deposition of about 200ppm or more after rinsed off from the hair.

35. The conditioning composition of Claim 34 which provides silicone deposition of about 400ppm or more after rinsed off from the hair.

36. A method of conditioning hair, the method comprising following steps:
(i) after shampooing hair, applying to the hair an effective amount of the conditioning composition of any of Claims 1 to 3 for conditioning the hair; and
(ii) then rinsing the hair.

## Patentansprüche

1. Konditionierungszusammensetzung, umfassend nach Gewicht:
(a) zu 0,01 % bis 6,0 % ein hydrophob modifiziertes vernetztes kationisches verdickendes Polymer;
(b) zu 0,1 % bis 8,0 % ein Tensidsystem, das ein kationisches Tensid und ein nichtionisches Tensid umfasst;
(c) zu 0,1 % bis 10 % eine Silikonverbindung; und
(d) einen wässrigen Träger.

2. Konditionierungszusammensetzung, umfassend nach Gewicht:
(a) zu 0,01 % bis 6,0 % ein hydrophob modifiziertes vernetztes kationisches verdickendes Polymer;
(b) zu 0,1 % bis 8,0 % ein Tensidsystem, das nichtionisches Tensid mit einem HLB-Wert von ungefähr 11 bis ungefähr 20 umfasst;
(c) zu 0,1 % bis 10 % eine Silikonverbindung; und
(d) einen wässrigen Träger.

3. Konditionierungszusammensetzung, umfassend nach Gewicht:
(a) zu 0,01 % bis 6,0 % ein hydrophob modifiziertes vernetztes kationisches verdickendes Polymer;
(b) zu 0,1 % bis 8,0 % ein Tensidsystem, ausgewählt aus der Gruppe, bestehend aus kationischem Tensid, nichtionischem Tensid und Mischungen davon;
(c) zu 0,1 % bis 10 % eine Silikonverbindung, wobei die Silikonverbindung aus jenen mit einer durchschnittlichen Teilchengröße von 300 nm oder weniger in der Zusammensetzung, jenen, die in der Zusammensetzung im Wesentlichen löslich sind, und Mischungen davon ausgewählt ist und wobei die im Wesentlichen lösliche Silikonverbindung ausgewählt ist aus der Gruppe, bestehend aus Aminosilikonen, Aminosilikoncopolyolen, hydrophob modifizierten Aminosilikoncopolyolen, hydrophob modifizierten Amidosilikoncopolyolen und Mischungen davon; und
(d) einen wässrigen Träger.

4. Konditionierungszusammensetzung nach einem der Ansprüche 1 bis 3, wobei die Zusammensetzung im Wesentlichen frei von einer wasserunlöslichen öligen Verbindung, ausgewählt aus Kohlenwasserstoffen, Fettverbindungen und Mischungen davon, ist.

5. Konditionierungszusammensetzung nach einem der Ansprüche 1 bis 3, wobei die Zusammensetzung transparent oder lichtdurchlässig ist.

6. Konditionierungszusammensetzung nach Anspruch 5, wobei die Zusammensetzung transparent ist.

7. Konditionierungszusammensetzung nach Anspruch 6, wobei die Zusammensetzung eine Durchlässigkeit von 50 % oder mehr hat.

8. Konditionierungszusammensetzung nach Anspruch 7, wobei die Zusammensetzung eine Durchlässigkeit von 65 % oder mehr hat.

9. Konditionierungszusammensetzung nach Anspruch 8, wobei die Zusammensetzung eine Durchlässigkeit von 80 % oder mehr hat.

10. Konditionierungszusammensetzung nach einem der Ansprüche 1 bis 3, wobei die Zusammensetzung eine Viskosität von 100 cP bis 100.000 cP und einen Strukturviskositätsindex von 30 oder mehr hat.

11. Konditionierungszusammensetzung nach Anspruch 10, wobei die Zusammensetzung eine Viskosität von 1.000 cP bis 50.000 cP und einen Strukturviskositätsindex von 50 oder mehr hat.

12. Konditionierungszusammensetzung nach Anspruch 11, wobei die Zusammensetzung eine Viskosität von 2.000 cP bis 50.000 cP und einen Strukturviskositätsindex von 70 oder mehr hat.

13. Konditionierungszusammensetzung nach Anspruch 12, wobei die Zusammensetzung eine Viskosität von 5.000 cP bis 20.000 cP und einen Strukturviskositätsindex von 90 oder mehr hat.

14. Konditionierungszusammensetzung nach einem der Ansprüche 1 bis 3, wobei das hydrophob modifizierte vernetzte kationische verdickende Polymer ein hydrophob modifiziertes vernetztes kationisches verdickendes Acrylatpolymer ist.

15. Konditionierungszusammensetzung nach einem der Ansprüche 1 bis 3, wobei das hydrophob modifizierte vernetzte kationische verdickende Polymer eine hydrophile Gruppe hat.

16. Konditionierungszusammensetzung nach Anspruch 15, wobei die hydrophile Gruppe eine Polyalkylenoxidgruppe ist.

17. Konditionierungszusammensetzung nach Anspruch 16, wobei das Alkylenoxid ausgewählt ist aus der Gruppe, bestehend aus Ethylenoxid, Propylenoxid und Mischungen davon.

18. Konditionierungszusammensetzung nach einem der Ansprüche 1 bis 3, wobei das Tensidsystem in der Zusammensetzung im Wesentlichen löslich ist.

19. Konditionierungszusammensetzung nach Anspruch 1 oder 3, wobei das nichtionische Tensid einen HLB-Wert von 8 bis 22 hat.

20. Konditionierungszusammensetzung nach entweder Anspruch 1 oder 3, wobei das nichtionische Tensid ausgewählt ist aus der Gruppe, bestehend aus Isoceteth-20, PPG-5-Ceteth-20, gehärtetem PEG-40-Rizinusöl, Polysorbat-20, Laureth-20, Ceteth-10, Ceteth-20 und Mischungen davon.

21. Konditionierungszusammensetzung nach Anspruch 1 oder 3, wobei das kationische Tensid ausgewählt ist aus der Gruppe, bestehend aus quartären Ammoniumsalzen mit einem langen Alkyl, quartären Ammoniumsalzen mit zwei langen Alkylketten, hydrophil substituierten quartären Ammoniumsalzen mit einem langen Alkyl, hydrophil substituierten quartären Ammoniumsalzen mit zwei langen Alkylketten, Aminen mit einer langen Alkylkette, Aminen mit zwei Alkylketten und Mischungen davon.

22. Konditionierungszusammensetzung nach Anspruch 21, wobei das kationische Tensid ausgewählt ist aus der Gruppe, bestehend aus Cetyltrimethylammoniumchlorid, Stearyltrimethylammoniumchlorid, Dicetyldimethylammoniumchlorid und Mischungen davon.

23. Konditionierungszusammensetzung nach Anspruch 1 oder 2, wobei die Silikonverbindung aus jenen mit einer durchschnittlichen Teilchengröße von 300 nm oder weniger in der Zusammensetzung, jenen, die in der Zusammensetzung im Wesentlichen löslich sind und Mischungen davon ausgewählt ist.

24. Konditionierungszusammensetzung nach Anspruch 23, wobei die im Wesentlichen lösliche Silikonverbindung ausgewählt ist aus der Gruppe, bestehend aus Aminosilikonen, Aminosilikoncopolyolen, hydrophob modifizierten Aminosilikoncopolyolen, hydrophob modifizierten Amidosilikoncopolyolen und Mischungen davon.

25. Konditionierungszusammensetzung nach Anspruch 24, wobei die Silikonverbindung ein hydrophob modifiziertes Amidosilikoncopolyol ist.

26. Konditionierungszusammensetzung nach einem der Ansprüche 1 bis 3, die ferner ein Hilfslösungsmittel umfasst.

27. Konditionierungszusammensetzung nach Anspruch 26, wobei das Hilfslösungsmittel ausgewählt ist aus der Gruppe, bestehend aus 1,2-Hexandiol, Hexylenglycol, Butylenglycol, Glycerin und Mischungen davon.

28. Konditionierungszusammensetzung nach einem der Ansprüche 1 bis 3, umfassend nach Gewicht:
(a) zu 0,1 % bis 5,0 % das hydrophob modifizierte vernetzte kationische verdickende Polymer;
(b) zu 0,2 % bis 5,0 % das Tensidsystem nach einem der Ansprüche 1 bis 3;
(c) zu 0,5 % bis 8,0 % die Silikonverbindung; und
(d) einen wässrigen Träger.

29. Konditionierungszusammensetzung nach Anspruch 28, umfassend nach Gewicht:
(a) zu 0,3 % bis 4,0 % das hydrophob modifizierte vernetzte kationische verdickende Polymer;
(b) zu 0,4 % bis 4,0 % das Tensidsystem nach einem der Ansprüche 1 bis 3;
(c) zu 1,0 % bis 6,0 % die Silikonverbindung; und
(d) einen wässrigen Träger.

30. Konditionierungszusammensetzung nach einem der Ansprüche 1 bis 3, die eine Haarkonditionierungszusammensetzung ist.

31. Konditionierungszusammensetzung nach einem der Ansprüche 1 bis 3, die zur Verwendung mit Abspülen dient.

32. Konditionierungszusammensetzung nach einem der Ansprüche 1 bis 3, die für eine Silikonanlagerung von ungefähr 50 ppm oder mehr nach dem Ausspülen aus dem Haar sorgt.

33. Konditionierungszusammensetzung nach Anspruch 32, die für eine Silikonanlagerung von ungefähr 100 ppm oder mehr nach dem Ausspülen aus dem Haar sorgt.

34. Konditionierungszusammensetzung nach Anspruch 33, die für eine Silikonanlagerung von ungefähr 200 ppm oder mehr nach dem Ausspülen aus dem Haar sorgt.

35. Konditionierungszusammensetzung nach Anspruch 34, die für eine Silikonanlagerung von ungefähr 400 ppm oder mehr nach dem Ausspülen aus dem Haar sorgt.

36. Verfahren zum Konditionieren von Haar, wobei das Verfahren die folgenden Schritte umfasst:
(i) nach dem Shamponieren des Haars Auftragen einer wirksamen Menge der Konditionierungszusammensetzung nach einem der Ansprüche 1 bis 3 auf das Haar zur Konditionierung des Haars; und
(ii) anschließendes Spülen des Haars.

## Revendications

1. Composition de conditionnement comprenant en poids :
(a) de 0,01 % à 6,0 % d'un polymère épaississant réticulé cationique rendu hydrophobe ;
(b) de 0,1 % à 8,0 % d'un système tensioactif comprenant un agent tensioactif cationique et un agent tensioactif non ionique ;
(c) de 0,1 % à 10 % d'un composé de silicone ; et
(d) un véhicule aqueux.

2. Composition de conditionnement comprenant en poids :
(a) de 0,01 % à 6,0 % d'un polymère épaississant réticulé cationique rendu hydrophobe ;
(b) de 0,1 % à 8,0 % d'un système tensioactif comprenant un agent tensioactif non ionique ayant un rapport hydro-lipophile allant d'environ 11 à environ 20 ;
(c) de 0,1 % à 10 % d'un composé de silicone ; et
(d) un véhicule aqueux.

3. Composition de conditionnement comprenant en poids :
(a) de 0,01 % à 6,0 % d'un polymère épaississant réticulé cationique rendu hydrophobe ;
(b) de 0,1 % à 8,0 % d'un système tensioactif choisi dans le groupe constitué d'agent tensioactif cationique, agent tensioactif non ionique, et leurs mélanges ;
(c) de 0,1 % à 10 % d'un composé de silicone, dans laquelle le composé de silicone est choisi parmi ceux ayant une taille moyenne des particules de 300 nm ou moins dans la composition, ceux étant essentiellement solubles dans la composition, et leurs mélanges, et dans laquelle le composé de silicone essentiellement soluble est choisi dans le groupe constitué d'aminosilicones, copolyols d'aminosilicone, copolyols d'aminosilicone rendus hydrophobes, copolyols d'amidosilicone rendus hydrophobes, et leurs mélanges ; et
(d) un véhicule aqueux.

4. Composition de conditionnement selon l'une quelconque des revendications 1 à 3, où la composition est essentiellement dépourvue d'un composé huileux insoluble dans l'eau choisi parmi les hydrocarbures, les composés gras, et leurs mélanges.

5. Composition de conditionnement selon l'une quelconque des revendications 1 à 3, où la composition est transparente ou translucide.

6. Composition de conditionnement selon la revendication 5, où la composition est transparente.

7. Composition de conditionnement selon la revendication 6, où la composition a une transmittance de 50 % ou plus.

8. Composition de conditionnement selon la revendication 7, où la composition a une transmittance de 65 % ou plus.

9. Composition de conditionnement selon la revendication 8, où la composition a une transmittance de 80 % ou plus.

10. Composition de conditionnement selon l'une quelconque des revendications 1 à 3, où la composition a une viscosité allant de 100 cP à 100 000 cP et un indice de rhéofluidification de 30 ou plus.

11. Composition de conditionnement selon la revendication 10, où la composition a une viscosité allant de 1000 cP à 50 000 cP et un indice de rhéofluidification de 50 ou plus.

12. Composition de conditionnement selon la revendication 11, où la composition a une viscosité allant de 2000 cP à 50 000 cP et un indice de rhéofluidification de 70 ou plus.

13. Composition de conditionnement selon la revendication 12, où la composition a une viscosité allant de 5000 cP à 20 000 cP et un indice de rhéofluidification de 90 ou plus.

14. Composition de conditionnement selon l'une quelconque des revendications 1 à 3, dans laquelle le polymère épaississant réticulé cationique rendu hydrophobe est un polymère épaississant acrylate cationique réticulé rendu hydrophobe.

15. Composition de conditionnement selon l'une quelconque des revendications 1 à 3, dans laquelle le polymère épaississant réticulé cationique rendu hydrophobe a un groupe hydrophile.

16. Composition de conditionnement selon la revendication 15, dans laquelle le groupe hydrophile est un groupe poly(oxyde d'alkylène).

17. Composition de conditionnement selon la revendication 16, dans laquelle l'oxyde d'alkylène est choisi dans le groupe constitué d'oxyde d'éthylène, oxyde de propylène, et leurs mélanges.

18. Composition de conditionnement selon l'une quelconque des revendications 1 à 3, dans laquelle le système tensioactif est essentiellement soluble dans la composition.

19. Composition de conditionnement selon la revendication 1 ou 3, dans laquelle l'agent tensioactif non ionique a un rapport hydro-lipophile allant de 8 à 22.

20. Composition de conditionnement selon l'une ou l'autre des revendications 1 ou 3, dans laquelle l'agent tensioactif non ionique est choisi dans le groupe constitué d'isocéteth-20, PPG-5-Céteth-20, huile de ricin hydrogénée PEG-40, polysorbate-20, lauréthyl-20, céteth-10, céteth-20, et leurs mélanges.

21. Composition de conditionnement selon la revendication 1 ou 3, dans laquelle l'agent tensioactif cationique est choisi dans le groupe constitué de sels d'ammonium quaternaire mono-alkyle long, sels d'ammonium quaternaire di-alkyle long, sels d'ammonium quaternaire mono-alkyle long à substitution hydrophile, sels d'ammonium quaternaire di-alkyle long à substitution hydrophile, amines à chaîne mono-alkyle longue, amine à chaîne di-alkyle, et leurs mélanges.

22. Composition de conditionnement selon la revendication 21, dans laquelle l'agent tensioactif cationique est choisi dans le groupe constitué de chlorure de cétyltriméthylammonium, chlorure de stéaryltriméthylammonium, chlorure de di-cétyl-diméthylammonium, et leurs mélanges.

23. Composition de conditionnement selon la revendication 1 ou 2, dans laquelle le composé de silicone est choisi parmi ceux ayant une taille moyenne des particules de 300 nm ou moins dans la composition, ceux étant essentiellement solubles dans la composition, et leurs mélanges.

24. Composition de conditionnement selon la revendication 23, dans laquelle le composé de silicone essentiellement soluble est choisi dans le groupe constitué d'aminosilicones, copolyols d'aminosilicone, copolyols d'aminosilicone rendus hydrophobes, copolyols d'amidosilicone rendus hydrophobes, et leurs mélanges.

25. Composition de conditionnement selon la revendication 24, dans laquelle le composé de silicone est un copolyol d'amidosilicone rendu hydrophobe.

26. Composition de conditionnement selon l'une quelconque des revendications 1 à 3, comprenant, en outre, un cosolvant.

27. Composition de conditionnement selon la revendication 26, dans laquelle le cosolvant est choisi dans le groupe constitué de 1,2-hexane diol, hexylène glycol, butylène glycol, glycérine et leurs mélanges.

28. Composition de conditionnement selon l'une quelconque des revendications 1 à 3, comprenant en poids :
(a) de 0,1 % à 5,0 % du polymère épaississant réticulé cationique rendu hydrophobe ;
(b) de 0,2 % à 5,0 % du système tensioactif selon l'une quelconque des revendications 1 à 3 ;
(c) de 0,5 % à 8,0 % du composé de silicone ; et
(d) un véhicule aqueux.

29. Composition de conditionnement selon la revendication 28, comprenant en poids :
(a) de 0,3 % à 4,0 % du polymère épaississant réticulé cationique rendu hydrophobe ;
(b) de 0,4 % à 4,0 % du système tensioactif selon l'une quelconque des revendications 1 à 3 ;
(c) de 1,0 % à 6,0 % du composé de silicone ; et
(d) un véhicule aqueux.

30. Composition de conditionnement selon l'une quelconque des revendications 1 à 3, qui est une composition de conditionnement des cheveux.

31. Composition de conditionnement selon l'une quelconque des revendications 1 à 3, qui est pour une utilisation à éliminer par rinçage.

32. Composition de conditionnement selon l'une quelconque des revendications 1 à 3, qui fournit un dépôt de silicone d'environ 50 ppm ou plus après avoir été enlevée par rinçage des cheveux.

33. Composition de conditionnement selon la revendication 32, qui fournit un dépôt de silicone d'environ 100 ppm ou plus après avoir été enlevée par rinçage des cheveux.

34. Composition de conditionnement selon la revendication 33, qui fournit un dépôt de silicone d'environ 200 ppm ou plus après avoir été enlevée par rinçage des cheveux.

35. Composition de conditionnement selon la revendication 34, qui fournit un dépôt de silicone d'environ 400 ppm ou plus après avoir été enlevée par rinçage des cheveux.

36. Procédé de conditionnement des cheveux, le procédé comprenant les étapes suivantes :
(i) après shampouinage des cheveux, application sur les cheveux d'une quantité efficace de la composition de conditionnement selon l'une quelconque des revendications 1 à 3 pour conditionner les cheveux ; et
(ii) puis rinçage des cheveux.
